# EUROPEAN PATENT APPLICATION

(11) **EP 3 944 256 A1**
(43) Date of publication of application: **26.01.2022**
(21) Application number: 20187256.1
(22) Date of filing: 22.07.2020
(51) Int. Cl.: G16H 50/20, G06N 3/08

(54) **METHODS AND SYSTEMS FOR UTILIZING AN ECG DATABASE**

(71) Applicant: Koninklijke Philips N.V., 5656 AG Eindhoven (NL)
(72) Inventor: JIN, Kim, 5656 AE Eindhoven (NL); GE, Essien, 5656 AE Eindhoven (NL)
(74) Representative: Philips Intellectual Property & Standards

(57) **Abstract**

The invention provides a method for generating a training set of data for training a classifier relating to a physiological condition. The method begins by obtaining a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features.

Descriptive statistics are calculated for each of the plurality of subsets of data within the first set of data and one or more features within the plurality of features is selected based on the calculated descriptive statistics to generate a search criterion. A supplementary set of data is identified from the second set of data by applying the search criterion to the second set of data. The training data set is then compiled based on the first set of data and the supplementary set of data.

## Description

### FIELD OF THE INVENTION

The invention relates to the field of data handling, and more specifically to the field of database searching.

### BACKGROUND OF THE INVENTION

Research toolsets can help physicians work more efficiently on clinical research. Diagnostic electrocardiogram (ECG) data is widely used for clinical diagnosis and screening and physicians require many different, advanced tools to help them work on ECG based research.

An ECG management system may be used to manage all ECG data within a given database and may include, or facilitate, a research platform and/or toolset on the in order to provide a convenient means of conducting ECG related research.

Typically, for applications requiring the use of classification algorithms, sets of data from two or more categories are needed for training a classifier that can then be used to classify new input data. The type of data used to train the classifier has a significant effect on the accuracy of the classifier.

There is therefore a need for a means of identifying and obtaining a desired type of data for training a classifier.

Further, one of the most important features of an ECG research toolset is a search function adapted to find data matching a given criteria. The search function is also usually the first module to be used in a research workflow for many research topics, as preparing data is typically the first step before subsequent processing. Accordingly, the search function plays a significant role in the research workflow as the data found using the search function will form the basis of the remaining research.

There is therefore a need for a means to search for data within a database to fulfil a given research requirement.

### SUMMARY OF THE INVENTION

The invention is defined by the claims.

According to examples in accordance with an aspect of the invention, there is provided a method for generating a training set of data for training a classifier relating to a physiological condition, the method comprising:
obtaining a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features,
calculating descriptive statistics for each of the plurality of subsets of data within the first set of data;
selecting one or more features within the plurality of features based on the calculated descriptive statistics to generate a search criterion;
identifying a supplementary set of data from the second set of data by applying the search criterion to the second set of data; and
compiling the training data set based on the first set of data and the supplementary set of data.

The method provides a means of identifying key features of interest in a first set of data, which may then be used to identify further supplementary data related to the features of interest. The first set of data and the supplementary data are then compiled into a training set of data related to the features of interest, thereby obtaining a training set that is specialized to train a classifier based on the features of interest. In this way, a classifier trained using the training set is adapted to obtain data and results related to the application of interest, such as a research project.

Put another way, the method provides for a means of customizing the compiling of a training data set for generating a specialized classifier that is trained for a given purpose.

In an embodiment, the first set of data comprises a first label indicating the presence of a physiological condition in the first plurality of subject and a second set of data comprises a second label indicating the absence of the physiological condition in the second plurality of subjects.

In this way, a training set may be compiled using both data associated with the presence of a physiological condition and data associated with the absence of a physiological condition that share similar features.

In an embodiment, the first set of data comprises one or more of:
a numerical value representing a measurement obtained from one of the first plurality of subjects;
a categorical value indicating a category of a measurement or a category of a statement relating to one of the first plurality of subjects;
and wherein, the step of calculating descriptive statistics comprises, for each of the plurality of subsets of data within the first set of data:
   for each subset of data comprising a numerical value, calculating at least one of a mean, a median, a standard deviation, a variance, a maximum and a minimum; or
   for the subset of data with a categorical value, calculating a percentage presence of each category within the subset of data.

In this way, the features of interest may be determined based on a measurement value or a categorical value, such as a statement or diagnosis.

In an embodiment, the method further comprises:
displaying the plurality of features and the calculated descriptive statistics corresponding to each of the plurality of features via a user interface; and
receiving a first user input by way of the user interface indicating one or more features of interest within the plurality of features.

In this way, the user may select the features of interest according to the desired application of the training set of data.

In a further embodiment, before the step of receiving the first user input, the method further comprises:
visualizing at least one subset of data within the first set of data and/or the corresponding calculated descriptive statistics associated with the at least one subset of data; and
displaying the visualization result via the user interface.

In this way, the descriptive statistics may more clearly be presented to the user in order to more readily identify potential features of interest to be selected.

In an embodiment, wherein the method further comprises:
displaying a template expression of the search criterion via a user interface;
receiving a second user input indicating an edit of the template expression to generate a search criterion based on the one or more features, the calculated descriptive statistics corresponding to the one or more features and the second user input.

In this way, the search criterion may be fine-tuned, thereby further increasing the control of the data compiled into the training set of data.

In an embodiment, the method further comprises applying an additional criterion to filter the supplementary data set.

In this way, potentially irrelevant data may be prevented from forming a part of the compiled training set of data.

According to examples in accordance with an aspect of the invention, there is provided a computer program comprising computer program code means which is adapted, when said computer program is run on a computer, to implement the methods described above.

According to examples in accordance with an aspect of the invention, there is provided a system for generating a training set of data for training a classifier relating to a physiological condition, the system comprising a processor adapted to:
obtain a first set of data relating to a first plurality of subjects and a second set of data relating to a second plurality of subjects, wherein each of the first set of data and the second set of data comprises a plurality of subsets of data, wherein the plurality of subsets of data is associated with a plurality of features,
calculate descriptive statistics for each of the plurality of subsets of data within the first set of data;
select one or more features within the plurality of features based on the calculated descriptive statistics to generate a search criterion;
identify a supplementary set of data from the second set of data by applying the search criterion to the second set of data; and
compile the training data set based on the first set of data and the supplementary set of data.

In an embodiment, the first set of data comprises a first label indicating the presence of a physiological condition in the first plurality of subject and a second set of data comprises a second label indicating the absence of the physiological condition in the second plurality of subjects.

In an embodiment, the first set of data comprises one or more of:
a numerical value representing a measurement obtained from one of the first plurality of subjects;
a categorical value indicating a category of a measurement or a category of a statement relating to one of the first plurality of subjects;
and wherein, when calculating the descriptive statistics the processor is adapted to, for each of the plurality of subsets of data within the first set of data:
   for each subset of data comprising a numerical value, calculate at least one of a mean, a median, a standard deviation, a variance, a maximum and a minimum; or
   for the subset of data with a categorical value, calculate a percentage presence of each category within the subset of data.

In an embodiment, the system further comprises a user interface adapted to:
display the plurality of features and the calculated descriptive statistics corresponding to each of the plurality of features; and
receive a first user input indicating one or more features of interest within the plurality of features.

In a further embodiment, before receiving the first user input, the processor is adapted to generate a visualization of at least one subset of data within the first set of data and/or the corresponding calculated descriptive statistics associated with the at least one subset of data, and wherein the user interface is further adapted to display the visualization result via the user interface.

In an embodiment, the system further comprises a user interface adapted to:
display a template expression of the search criterion;
receive a second user input indicating an edit of the template expression to generate a search criterion based on the one or more features, the calculated descriptive statistics corresponding to the one or more features and the second user input.

In an embodiment, the processor is further adapted to apply an additional criterion to filter the supplementary data set.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for generating a training set of data according to an aspect of the invention;
Figure 2 shows a schematic representation of a user interface;
Figures 3a and 3b show a schematic representation of an example of a user interface according to an aspect of the invention; and
Figure 4 shows a schematic representation of an example of a user interface according to an aspect of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for generating a training set of data for training a classifier relating to a physiological condition. The method begins by obtaining a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features.

Descriptive statistics are calculated for each of the plurality of subsets of data within the first set of data and one or more features within the plurality of features is selected based on the calculated descriptive statistics to generate a search criterion. A supplementary set of data is identified from the second set of data by applying the search criterion to the second set of data. The training data set is then compiled based on the first set of data and the supplementary set of data.

A further aspect of the invention provides a system for searching a database of ECG data. The system includes a user interface adapted to receive a user input from a user and a processor.

The systems discussed herein may be implemented as part of any suitable processing system. The methods discussed herein may be performed using any suitable processing system.

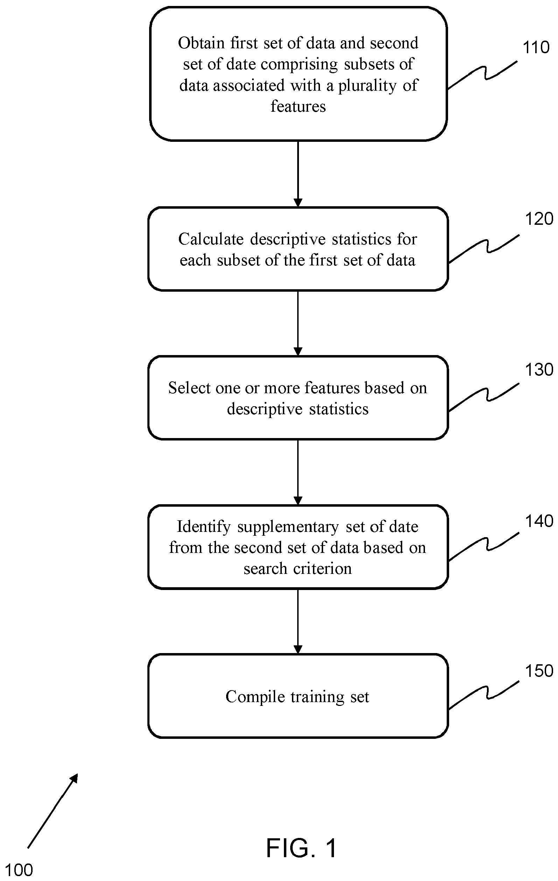

Figure 1 shows a method 100 for generating a training set of data for training a classifier relating to a physiological condition. The physiological condition may be any condition of a subject, such as a previously known diagnosed condition or a previously unknown condition, which may for example be defined by way of one or more symptoms. For the purposes of illustration, the methods described below refer to the use of ECG data; however, the principles described herein may be applied to any clinically relevant data.

The method begins in step 110 by obtaining a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features.

For example, in a typical research program, a physician may collect several special cases that require further investigation as part of the research, which are then treated as the first set of data. For example, the first set of data may include data relating to a first plurality of subjects with ECG measurement values as the data, all having a certain disease or cardiac abnormality. The first set of data comprises a plurality of subsets of data associated with a plurality of features.

By way of example, table 1 below provides an example of a first set of data, wherein each row represents a different subject and each column represents a different subset of data corresponding to a feature of the first set of data. Put another way, all of the data points in each column of the table below share a common feature and form a subset of data when grouped together.

**Table 1: An example of a first set of data comprising a plurality of features represented in the columns**

| Subject | Subject Statement | Ramp@I | Ramp@II | Ramp@III | Ramp@IV | Ramp@V |
|---|---|---|---|---|---|---|
| 1 | AGMUNK | 655 | 565 | 27 | 485 | 35 |
| 2 | SR RBBB | 413 | 278 | 55 | 322 | 199 |
| 3 | SR AMIAD | 521 | 377 | 77 | 480 | 0 |
| 4 | AGMUNK | 0 | 567 | 1530 | 0 | 191 |
| 5 | AGMUNK | 834 | 1211 | 950 | 594 | 0 |

In the example shown in table 1, ramp@N means R wave amplitude value at Lead N in an ECG waveform, i.e. ramp@I refers to R wave amplitude at Lead 1. Typically, there are 12 leads within an ECG wave, wherein the term lead refers to a line defined between two electrodes along which the signal is measured. Each piece of data in the table is taken from an ECG waveform obtained from a subject and calculated by way of an algorithm. The algorithm may extract a plurality of features from the ECG waveform, such as amplitude of a wave or time interval between waves.

If the data includes a categorical value, for example a statement, such as a diagnosis or symptom, the user may be provided with a descriptive statistic indicating, for example, the data sharing the most frequent usage of the statement. For example, in table 1, AGMUNK may represent that the age and gender of the subject in that row is unknown. Further, SR may indicate that the Sinus rhythm is of interest, RBBB may indicate a right bundle-branch block and AMIAD may indicate an acute anterior infarction. Statements such as these may act as features in order to identify data of interest.

The first set of data may comprise a first label indicating the presence of a physiological condition in the first plurality of subjects and a second set of data comprises a second label indicating the absence of the physiological condition in the second plurality of subjects.

The inventors have recognized that after researchers have obtained a first set of data with a positive label class, data with a negative label class may be prepared based on an assessment of the first set of data to improve subsequent machine learning based analyses.

In step 120, descriptive statistics are calculated for each of the plurality of subsets of data within the first set of data.

For example, the first set of data may comprise one or more of: a numerical value representing a measurement obtained from one of the first plurality of subjects; a categorical value indicating a category of a measurement or a category of a statement relating to one of the first plurality of subjects.

The step of calculating descriptive statistics may then comprise, for each of the plurality of subsets of data within the first set of data: for each subset of data comprising a numerical value, calculating at least one of a mean, a median, a standard deviation, a variance, a maximum and a minimum; or for the subset of data with a categorical value, calculating a percentage presence of each category within the first set of data.

In the example, provided above in Table 1, the first set of data comprises both numerical values representing measurements obtained from the first plurality of subjects and categorical values in the statement column

**Table 2: An example of a first set of data comprising a plurality of features represented in the columns and associated descriptive statistics**

| Subject | Subject Statement | Ramp@I | Ramp@II | Ramp@III | Ramp@IV | Ramp@V |
|---|---|---|---|---|---|---|
| 1 | AGMUNK | 655 | 565 | 27 | 485 | 35 |
| 2 | SR RBBB | 413 | 278 | 55 | 322 | 199 |
| 3 | SRAMIAD | 521 | 377 | 77 | 480 | 0 |
| 4 | AGMUNK | 0 | 567 | 1530 | 0 | 191 |
| 5 | AGMUNK | 834 | 1211 | 950 | 594 | 0 |
| Variance | | 78481 | 105809.4 | 372043.8 | 42902.6 | 8236.4 |

Table 2 shows the data of Table 1 with the addition of the variance of each column in the final row of the table as a descriptive statistic for each feature. The variance may be replaced by any suitable descriptive statistic. Further, the categorical values, in the form of the statements in the statement column, may be used to generate descriptive statistics, such as a rate of occurrence of a given statement. By way of example, in Table 2, the statement AGMUNK occurs in 60% of subjects.

In step 130, one or more features within the plurality of features are selected based on the calculated descriptive statistics to generate a search criterion.

The search criterion may comprise one or more of: equal to the mean; not equal to the mean; greater than the mean; less than the mean; and the like. The selection of the one or more features may be performed automatically, for example, based on a known relationship between features or a detected anomaly in a descriptive statistic, or manually by way of a user input.

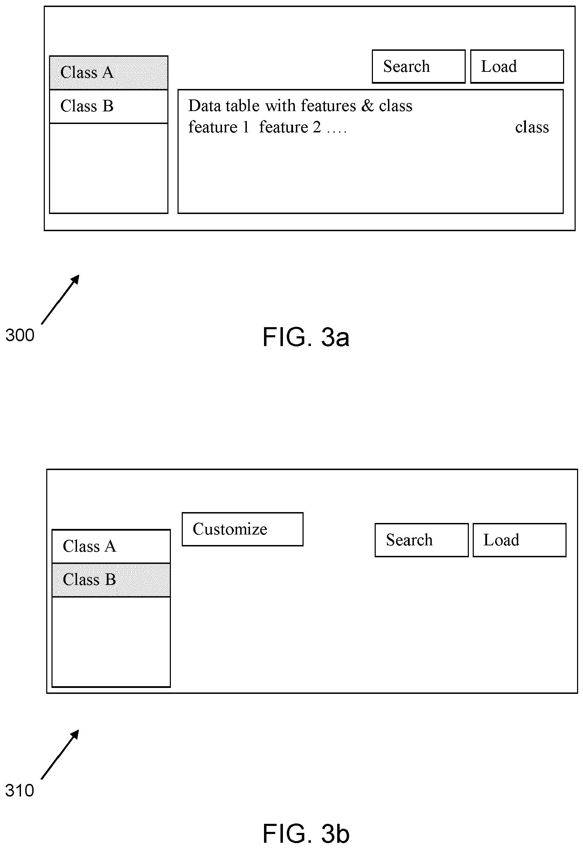

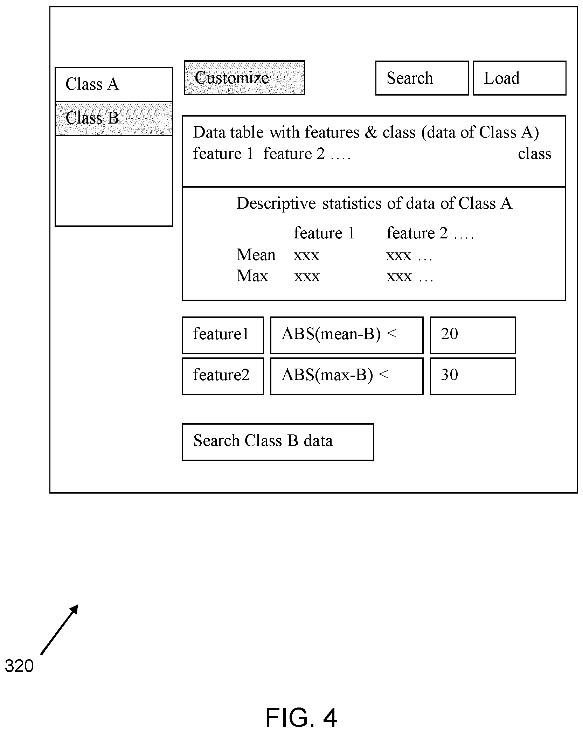

For example, the plurality of features and the calculated descriptive statistics corresponding to each of the plurality of features may be displayed to a user by way of a user interface, an example of which is described further below with reference to Figures 3a, 3b and 4. A first user input may then be received by way of the user interface indicating one or more features of interest within the plurality of features. In this way, the user may direct the generation of the search criterion in order to obtain supplementary data of interest from the second set of data.

Further, a template expression of the search criterion may be displayed to the user by way of the user interface and a second user input may be received indicating an edit of the template expression to generate a search criterion based on the one or more features, the calculated descriptive statistics corresponding to the one or more features and the second user input.

Put another way, before being finalized, the search criterion may be presented to a user for the purpose of editing the search criterion according to the desired supplementary data.

At this stage of the method, the second set of a data is an unrefined dataset, which may simply be the remaining data after the first set of data has been selected from a generic database. For example, a database may comprise every subject, for example from a given hospital or clinic, that has had ECG data collected from them. In an exemplary implementation, when those subjects that have a given cardiac irregularity have been assigned a positive label and separated into the first set of data, the second set of data may be those subjects remaining.

In step 140, a supplementary set of data is identified from the second set of data by applying the search criterion to the second set of data. The identified supplementary set of data may be further filtered by applying an additional criterion to filter the supplementary data set. The additional criterion may include: age; demographic; gender; and the like.

In step 150, the training data set is compiled based on the first set of data and the supplementary set of data.

A classifier may then be trained based on the complied data. A classifier is a type of machine learning algorithm. A machine-learning algorithm is any self-training algorithm that processes input data in order to produce or predict output data. Here, the input data comprises the compiled data and the output data comprises the classification of the classifier.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example compiled data from the first set of data and the relevant supplementary data. The training output data entries correspond to classifications.

In other words, the proposed method will first analyze the first set of data and show descriptive analysis results for the selection of certain conditions for the second set of data, which will then be searched to extract the relevant supplementary data, for example from an ECG data management system.

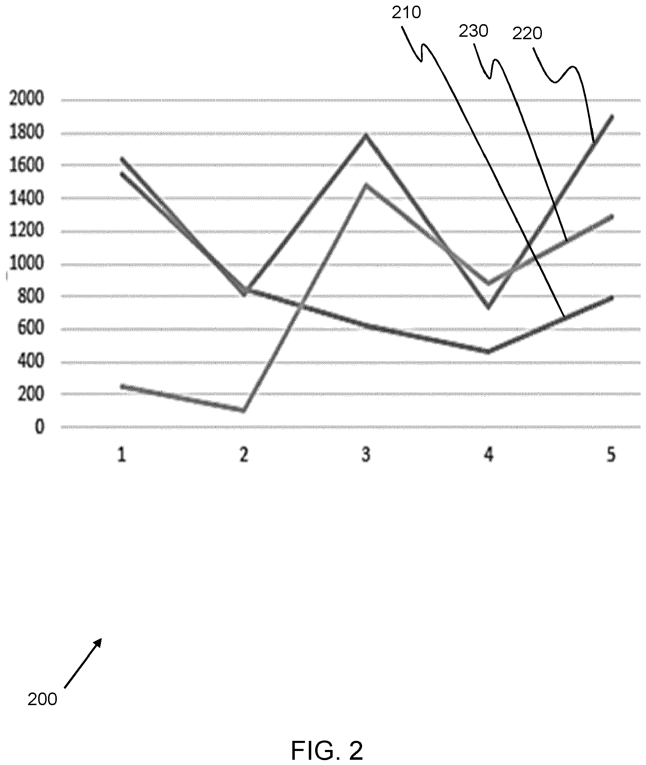

Figure 2 shows a visualization 200 of a plurality of subsets of data within the first set of data shown in Table 3 below.

**Table 3: An example of a first set of data comprising a plurality of features represented in the columns with the data visualized in Figure 2 highlighted**

| Subject | Subject Statement | Ramp@I | Ramp@II | Ramp@III |
|---|---|---|---|---|
| 1 | ST LVHSR | 1553 | 1634 | 251 |
| 2 | AGMUNK | 846 | 819 | 106 |
| 3 | AGMUNK | 642 | 1781 | 1478 |
| 4 | SB APC LVHSR | 460 | 737 | 878 |
| 5 | AGMUNK | 796 | 1896 | 1290 |

In the graph shown in Figure 2, the plots represent the measurement data for ramp@1 with plot line 210, ramp@2 with plot line 220 and ramp@3 with plot line 230. In use, and before the step of receiving the first user input, a subset of data within the first set of data and/or the corresponding calculated descriptive statistics associated with the at least one subset of data may be visualized and displayed by way of a user interface. Examples of descriptive statistics that may be derived from the data of Table 3 are shown below in Table 4.

**Table 4: An example descriptive statistics that may be derived from the data of Table 3**

| | ramp@I | ramp@II | ramp@III |
|---|---|---|---|
| count | 5.000000 | 5.000000 | 5.000000 |
| mean | 855.800000 | 1373.400000 | 800.600000 |
| std | 418.374473 | 552.160574 | 610.103106 |
| min | 460.000000 | 737.000000 | 106.000000 |
| 25% | 624.000000 | 819.000000 | 251.000000 |
| 50% | 796.000000 | 1634.000000 | 878.000000 |
| 75% | 846.000000 | 1781.000000 | 1290.000000 |
| max | 1553.000000 | 1896.000000 | 1478.000000 |

The descriptive statistics shown in Table 4 include: count, which represents the number of data points in the subset of data; mean, which represents the mean of the subset of data; std, which represents the standard deviation of the subset of data; min, which represents the minimum value of the subset of data; max, which represents the maximum value of the subset of data; 25%, 50% and 75%, which represent the first, second and third quartiles of the data, respectively.

Figures 3a and 3b provide schematic representations of a user interface implementing a worked example of the methods described above. Figure 3a shows a schematic representation of a user interface 300, which may implemented as part of a system for performing the methods described above.

In Figure 3a the first button, Class A, represents the first set of data, which comprises data from the first plurality of subjects, and the second button, Class B, represents the second set of data, which comprises data from the second plurality of subjects. Further, the user interface shows a data table of the data corresponding to the selected set of data, which is the first set of data in the example shown in Figure 3a by way of highlighting the button Class A, wherein the data table comprises data divided into columns corresponding to a plurality of features (feature 1, feature 2 and the like).

During use, the user may select the first set of data to get data into the data table. For example, the user may select the Search or Load buttons shown in Figure 3a in order to bring data into the table. The user may then select the second button, as shown in the example 310 in Figure 3b, and be presented with a prompt to provide an indication as to how the second set of data should be filled. For example, the user may be presented with a Customize button, which may be used to initiate obtaining supplementary data from the second set of data.

Figure 4 shows an example 320 of the schematic representation of the user interface shown in Figures 3a and 3b during the process of obtaining supplementary data from the first set of data.

In the example shown in Figure 4, the system first calculates descriptive statistics of the first set of data to provide for the user's reference. The descriptive statistics results may include mean, standard deviation, minimum and maximum values, and the like, of the features of the first set of data. The descriptive statistics may be shown in a table as shown in Figure 4. The column(s) of features in the table may be selected by the user to indicate features of interest.

Put another way, the descriptive statistics of the features shown in the table may be displayed to the user by way of the interface. The descriptive statistics may serve to provide the user with additional information for selecting the features of interest. A further visualization of the descriptive statistics may be provided to the user as described above with reference to Figure 2.

The user may select the features of interest according to how the user wants to select the data from the second set of data. For example, a selected column (corresponding to a feature) may form part of a condition formula for selecting relevant data from the second set of data. In the example shown in Figure 4, the features of interest are feature 1 and feature 2 and the condition formula states that for feature 1, the absolute value of the mean of the supplementary data from the second set of data must be less than 20 (feature1 ABS(mean-B)< 20) and for feature 2, the absolute value of the maximum value of the supplementary data from the second set of data must be less than 30 (feature 2 ABS(max-B)< 30). The user may customize the parameters of the condition accordingly. Further, multiple selected columns will result in mapping the condition formula to multiple conditions and the logic relationship of these conditions may also be adjusted by the user, or automatically.

After the conditions are set, the condition function may be used as a search criterion and the user may initiate a search of the second set of data, for example by selecting the Search Class B data button. An internal mechanism may then search the data from the ECG management system database that fulfils the conditions of the search criterion. Following the search, the obtained relevant data may be shown in a table on the user interface.

The user interface may comprise further elements to provide a means to further filter the searched data. For example, the method may include filtering the first set of data and the relevant supplementary data to make the data with positive and negative class labels have similar statistical distributions on certain features. For example, the age or gender for both groups may be restricted to obtain a similar distribution. In this way, it is possible to avoid the final classification result being interfered with by factors that are not of interest to the given research. The user may select which features should have similar statistical distributions and the system may automatically calculate the distribution by adding or removing data from the first or second sets of data.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (100) for generating a training set of data for training a classifier relating to a physiological condition, the method comprising:
obtaining (110) a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features;
calculating (120) descriptive statistics for each of the plurality of subsets of data within the first set of data;
selecting (130) one or more features within the plurality of features based on the calculated descriptive statistics to generate a search criterion;
identifying (140) a supplementary set of data from the second set of data by applying the search criterion to the second set of data; and
compiling (150) the training data set based on the first set of data and the supplementary set of data.

2. The method (100) as claimed in claim 1, wherein the first set of data comprises a first label indicating the presence of a physiological condition in the first plurality of subject and a second set of data comprises a second label indicating the absence of the physiological condition in the second plurality of subjects.

3. The method (100) as claimed in any of claims 1 to 2, wherein the first set of data comprises one or more of:
a numerical value representing a measurement obtained from one of the first plurality of subjects;
a categorical value indicating a category of a measurement or a category of a statement relating to one of the first plurality of subjects;
and wherein, the step of calculating descriptive statistics comprises, for each of the plurality of subsets of data within the first set of data:
for each subset of data comprising a numerical value, calculating at least one of a mean, a median, a standard deviation, a variance, a maximum and a minimum; or
for the subset of data with a categorical value, calculating a percentage presence of each category within the subset of data.

4. The (100) method as claimed in any of claims 1 to 3, wherein the method further comprises:
displaying the plurality of features and the calculated descriptive statistics corresponding to each of the plurality of features via a user interface; and
receiving a first user input by way of the user interface indicating one or more features of interest within the plurality of features.

5. The (100) method as claimed in claim 4, wherein, before the step of receiving the first user input, the method further comprises:
visualizing at least one subset of data within the first set of data and/or the corresponding calculated descriptive statistics associated with the at least one subset of data; and
displaying the visualization result via the user interface.

6. The (100) method as claimed in any of claims 1 to 5, wherein the method further comprises:
displaying a template expression of the search criterion via a user interface;
receiving a second user input indicating an edit of the template expression to generate a search criterion based on the one or more features, the calculated descriptive statistics corresponding to the one or more features and the second user input.

7. A (100) method as claimed in claims 1 to 6 wherein the method further comprises applying an additional criterion to filter the supplementary data set.

8. A computer program comprising computer program code means which is adapted, when said computer program is run on a computer, to implement the method of any of claims 1 to 7.

9. A system for generating a training set of data for training a classifier relating to a physiological condition, the system comprising a processor adapted to:
obtain a first set of data relating to a first plurality of subjects and a second set of data relating to a second plurality of subjects, wherein each of the first set of data and the second set of data are grouped into a plurality of subsets of data, wherein the plurality of subsets of data is associated with a plurality of features,
calculate descriptive statistics for each of the plurality of subsets of data within the first set of data;
select one or more features within the plurality of features based on the calculated descriptive statistics to generate a search criterion;
identify a supplementary set of data from the second set of data by applying the search criterion to the second set of data; and
compile the training data set based on the first set of data and the supplementary set of data.

10. The system as claimed in claim 9, wherein the first set of data comprises a first label indicating the presence of a physiological condition in the first plurality of subject and a second set of data comprises a second label indicating the absence of the physiological condition in the second plurality of subjects.

11. The system as claimed in any of claims 9 to 10, wherein the first set of data comprises one or more of:
a numerical value representing a measurement obtained from one of the first plurality of subjects;
a categorical value indicating a category of a measurement or a category of a statement relating to one of the first plurality of subjects;
and wherein, when calculating the descriptive statistics the processor is adapted to, for each of the plurality of subsets of data within the first set of data:
for each subset of data comprising a numerical value, calculate at least one of a mean, a median, a standard deviation, a variance, a maximum and a minimum; or
for the subset of data with a categorical value, calculate a percentage presence of each category within the subset of data.

12. The system as claimed in any of claims 9 to 11, wherein the system further comprises a user interface adapted to:
display the plurality of features and the calculated descriptive statistics corresponding to each of the plurality of features; and
receive a first user input indicating one or more features of interest within the plurality of features.

13. The system as claimed in claim 12, wherein, before receiving the first user input, the processor is adapted to generate a visualization of at least one subset of data within the first set of data and/or the corresponding calculated descriptive statistics associated with the at least one subset of data, and wherein the user interface is further adapted to display the visualization result via the user interface.

14. The system as claimed in any of claims 9 to 13, wherein the system further comprises a user interface adapted to:
display a template expression of the search criterion;
receive a second user input indicating an edit of the template expression to generate a search criterion based on the one or more features, the calculated descriptive statistics corresponding to the one or more features and the second user input.

15. A system as claimed in any of claims 9 to 14 wherein the processor is further adapted to apply an additional criterion to filter the supplementary data set.
